# EUROPÄISCHE PATENTSCHRIFT

(11) **EP 2 655 493 B1**
(45) Veröffentlichungstag und Bekanntmachung des Hinweises auf die Patenterteilung: **17.10.2018**
(21) Anmeldenummer: 11784471.2
(22) Anmeldetag: 11.11.2011
(51) Int. Cl.: B29B 7/74, C07C 4/22, C08J 11/12, C10G 1/02, C10G 1/10

(54) **VERFAHREN ZUR HERSTELLUNG UMWELTFREUNDLICHER WEICHMACHER**
PROCESS FOR MAKING ENVIROMENTAL FRIENDLY PLASTICIZERS
PROCÉDÉ DE PRODUCTION DE PLASTIFIANTS RESPECTUEUX DE L'ENVIRONNEMENT

(30) Priorität: 22.12.2010 DE 102010061480
(43) Veröffentlichungstag der Anmeldung: 30.10.2013
(73) Patentinhaber: Continental Reifen Deutschland GmbH, 30165 Hannover (DE)
(72) Erfinder: RECKER, Carla, 30167 Hannover (DE); HAAS, Ewgeni, 25479 Hamburg (DE)
(74) Vertreter: Preusser, Andrea
(86) Internationale Anmeldenummer: PCT/EP2011/069925
(87) Internationale Veröffentlichungsnummer: WO 2012/084335

(56) Entgegenhaltungen:
- EP-A1- 1 632 546
- WO-A1-2010/012531
- DE-A1- 10 215 679

## Beschreibung

Die Erfindung betrifft ein Verfahren zur Herstellung von umweltfreundlichem Weichmacher, welcher insbesondere zur Verwendung in Fahrzeugreifen oder technischen Gummiartikeln geeignet ist.

Neben Kautschuk und Füllstoffen bilden die Weichmacher eine andere wichtige Klasse von Zuschlagstoffen in Kautschukmischungen, wie sie für Fahrzeugreifen (Fahrzeugluftreifen, Zweiradreifen, Vollgummireifen) und technischen Gummiartikeln (Schläuche, Riemen, Gurte, Drucktücher, Membranen, Dichtungen, Luftfedern, Bälge, Automobilinnenverkleidungen, Kunstleder) verwendet werden. Weichmacher werden Kautschukmischungen zum Teil in großen Mengen zugesetzt, um dem Mischungspreis zu senken, die Fließeigenschaften der Mischung zu verbessern (Energieeinsparung bei der Verarbeitung, Vermeidung von Energiespitzen), die Füllstoffdispersion zu verbessern, das Konfektionier- und das Klebverhalten zu verbessern und die physikalischen Eigenschaften der Mischung und der daraus hergestellten Vulkanisate zu beeinflussen.

In der Regel ist die Basis fast aller in der Kautschukindustrie verwendeten Weichmacher Erdöl. Aus ökologischen Gesichtspunkten, vor allem im Bezug auf die gegebenen Schadstoffemissionen und Rohstoffknappheiten, ist Erdöl zukünftig als Ausgangsstoff für die Herstellung von Kautschuk-Weichmachern nicht mehr tragbar. Als Alternative werden beispielsweise Pflanzenöle als Weichmacher in Kautschukmischungen verwendet, wie beispielsweise in DE 101 08 981 A1 und DE 602 18 446 T2 beschrieben. Die dort beschriebenen Pflanzenöle können als alleiniger Weichmacher eingesetzt werden, meist werden sie aber in Kombination mit einem weiteren, aus Erdöl gewonnenen, Weichmacher verwendet. Aber auch die Pflanzenöle stehen allerdings nicht in beliebigen Mengen für die Kautschukindustrie zur Verfügung.
Als weitere Alternative wird in WO 2010/012531 A1 die Verwendung von sogenannten BTL-Ölen (Biomass-To-Liquid-Ölen) beschrieben. Hierbei sind feste Biomassen Ausgangstoff für die Herstellung der Weichmacher, die wiederum über verschiedene Arten erfolgen kann. Zu erwähnen sind hier beispielhaft die Flash-Pyrolyse, mit sehr kurzen Verweilzeiten im Reaktor, die hydrierende Direktverflüssigung, wo durch (Druck-) Wasserstoff während der Pyrolyse stabile Produktkohlenwasserstoffe entstehen, das so genannte Carbo-V-Verfahren, welches an das Fischer-Tropsch-Verfahren angelehnt ist und die katalytische Direktverflüssigung, bei der die Pyrolyse in einem Ölsumpf mit Katalysatorbeimengung erfolgt. Verfahren zur Direktverflüssigung sind sehr allgemein beispielsweise in DE 102 15 679 A1 und DE 10 2005 040 490 A1 beschrieben.
Ein Verfahren zur Direktverflüssigung von Biomassen ist aus Willner, Marktfruchtreport 2005, Mitteilungen der Landwirtschaftskammer, Landwirtschaftskammer Schleswig-Holstein, Kiel, bekannt.
Das Problem hierbei ist allerdings, dass die verfügbare Biomasse begrenzt ist. Wird die BTL-Herstellung, sowohl für Kraftstoffe als auch für Weichmacheröle, daher zukünftig in größerem Maßstab eingesetzt, so führt dies zu einer Flächen- und Nutzungskonkurrenz bedingt durch die erhöhte Nutzung von landwirtschaftlichen und forstwirtschaftlichen Flächen. Ebenso ist der Aufwand für Ernte, Transport, Schreddern und anderes zu berücksichtigen.

Der Erfindung liegt nun die Aufgabe zu Grunde, ein Verfahren zur Herstellung von umweltfreundlichen Weichmachern bereitzustellen, durch den ein Weichmacher erhalten wird, der zum Einen eine Unabhängigkeit gegenüber Erdöl als Rohstoffquelle und Energiequelle gewährleisten kann und zum Anderen die oben genannten Probleme, welche BTL-Öle bereiten, nicht aufweist. Des Weiteren sollen die Ausgangsstoffe für die Herstellung des Weichmachers relativ einfach verfügbar sein.

Gelöst wird diese Aufgabe durch ein Verfahren zur Herstellung von Weichmacher, welches dadurch gekennzeichnet ist, dass hochmolekulares vulkanisiertes Polymermaterial durch thermische Direktverflüssigung in eine niedrigmolekulare Phase umgewandelt wird.

Überraschenderweise wurde gefunden, dass sich ein umweltfreundlicher Weichmacher aus Polymermaterial herstellen lässt.
Hierzu wird das hochmolekulare vulkanisierte Polymermaterial durch thermische Direktverflüssigung in eine niedrigmolekulare Phase umgewandelt. Nach der Umwandlung wird die niedrigmolekulare Phase auf geeignetem und dem der fachkundigen Person bekannten Weg gewonnen und kann als umweltfreundlicher Weichmacher für Kautschukmischungen verwendet werden. Dies führt letztendlich zu einer deutlichen Verbesserung der Ökobilanz der Endprodukte, wie Fahrzeugreifen und technische Gummiartikel.
Das Vorliegen als niedrigmolekulare Phase bedeutet, dass der Weichmacher als Weichmacheröl vorliegt.
Bei dem hochmolekularen vulkanisierten Polymermaterial handelt es bevorzugt um Kautschuke, d.h. elastische Polymere, Kautschukmischungen (grün oder vulkanisiert), thermoplastische Elastomere (TPE), Mischungen, welche TPE enthalten (grün oder vulkanisiert), thermoplastische oder duroplastische Kunststoffe. Insbesondere Kautschukmischungen, welche zur Verwendung in Reifen oder technischen Gummiartikeln vorgesehen sind (grüne Mischung), vorgesehen waren (vulkanisierte Mischung, Abfälle) oder bereits hierfür verwendet wurden (vulkanisierte Mischung, Altabfälle) können zur Herstellung des Weichmachers eingesetzt werden. Gemische aus zwei oder mehr der oben genannten Polymermaterialien sind möglich.
Da es sich bei den genannten Polymermaterialien in der Regel um Abfallstoffe handelt, welche z.Bsp. während oder nach der Produktion von Reifen oder technischen Gummiartikeln (TRG = technical rubber goods) anfallen, welche als Altreifen oder Alt-TRGs vorliegen oder welche als Kunststoffabfälle bspw aus Verpackungen oder ähnlichem, bspw. Schredderleichtfraktionen, vorliegen, wird durch das erfindungsgemäße Verfahren ein zusätzlicher Verwertungsweg für derartige Abfallstoffe geschaffen.

Der durch thermische Direktverflüssigung von Polymermaterial hergestellte Weichmacher kann wie ein bisher in der Kautschukindustrie verwendeter Weichmacher eingesetzt werden. Es findet dabei ein echtes "Recycling" statt und kein "Downcycling", da in den fertigen Endprodukten keine Kompromisse hinsichtlich der physikalischen Eigenschaften eingegangen werden müssen.
Dies liegt insbesondere im Fall von füllstoffhaltigen Polymermaterialien auch daran, dass durch die Verwendung der thermischen Direktverflüssigung von Polymermaterial der so hergestellte Weichmacher keinen negativen Einfluss auf die Verstärkungswirkung der üblicherweise in Kautschukmischungen enthaltenen Füllstoffe hat. Bekannte pyrolytische Verfahren führen zu Weichmachern, die in der energetischen Verwertung als Brennstoff eingesetzt werden und wodurch in der Kautschukmischung enthaltene Füllstoffe ihr Verstärkungspotential deutlich verlieren.

Im Hinblick auf eine vereinfachte Prozessführung hat es sich als vorteilhaft gezeigt, wenn das Polymermaterial in Form von Polymerpulver und / oder Polymergranulat vorliegt. Dies führt zu einer vereinfachten Zuführung des Polymermaterials in die entsprechende Reaktionsapparatur.
Das Polymerpulver und / oder das Polymergranulat ist bevorzugt auf der Basis von Altreifen und enthält in einer bevorzugten Ausführungsform natürlichen oder synthetischen Kautschuk und / oder Butadienkautschuk und / oder Styrolbutadienkautschuk.
Diese Dienkautschuke werden üblicherweise zur Herstellung von Reifen und TRG verwendet und sind daher in den entsprechenden Abfallstoffen zu finden. Des Weiteren lässt sich bei Vorhandensein von den genannten Kautschuken im Polymermaterial eine besonders gute Ausbeute an Weichmacher erzielen. Bevorzugt zeigen sich Ausbeuten von 90% oder mehr.
Die thermische Direktverflüssigung von Polymermaterial findet im Wesentlichen in Anlehnung an die BTL-Herstellung statt.
Besonders gute Ergebnisse werden erzielt, wenn die thermische Direktverflüssigung von Polymermaterial bei Temperaturen zwischen 100 und 500°C, bevorzugt bei Temperaturen zwischen 150 und 420°C, stattfindet.

Vorteilhaft ist es ebenso, wenn das hochmolekulare vulkanisierte Polymermaterial in einer Sumpfphase gecrackt wird.
Die gecrackte Sumpfphase kann in einer bevorzugten Ausführungsform das Endprodukt darstellen.
Als Cracken bezeichnet die fachkundige Person die Spaltung von Kohlenwasserstoffen längerer Kettenlänge (hochmolekular) in Kohlenwasserstoffe kürzerer Kettenlänge (niedermolekular, auch als niedrigmolekular bezeichnet).

Bei der BTL-Herstellung wird oft in einstufige und zweistufige Verfahren unterschieden, wobei bei zweistufigen Verfahren im Herstellungsprozess im Wesentlichen in einem ersten Verfahrensschritt die Erzeugung eines Synthesegases mittels Vergasung und in einem zweiten Verfahrensschritt die Synthetisierung eines Treibstoffes erfolgt.
Im Rahmen des erfindungsgemäßen Verfahrens hat es sich als vorteilhaft gezeigt, wenn das Cracken einstufig stattfindet. Das einstufige Verfahren liefert eine gute Ausbeute und spart gleichzeitig einen weiteren Verfahrensschritt ein.
Des Weiteren kann das Verfahren absatzweise, d.h. schrittweise, oder kontinuierlich durchgeführt werden, wobei letztere die besonders bevorzugte Variante ist, da sie im industriellen Maßstab am wenigsten aufwendig ist.

Ebenso hat es sich als vorteilhaft gezeigt, wenn zur Umwandlung des hochmolekularen vulkanisierten Polymermaterials in eine niedrigmolekulare Phase wenigstens ein Vorlageöl verwendet wird. Dies gilt sowohl für das absatzweise Verfahren als auch für das kontinuierliche Verfahren, wobei beim kontinuierlichen Verfahren das Vorlageöl im Wesentlichen zum Anfahren des Prozesses verwendet wird.
Zunächst wird bei Verwendung eines Vorlageöls selbiges auf eine für das entsprechende Öl geeignete Temperatur erwärmt und anschließend wird das zu crackende Polymermaterial hinzugefügt. Eine geeignete Temperatur für das Vorlageöl sollte in der Regel eine Temperatur sein, bei der sich das Vorlageöl noch nicht vollständig anfängt zu zersetzen, was durch das Nicht-Vorhandensein eines Blindstroms zeigt.

In einer bevorzugten Ausführungsform ist das Vorlageöl ausgewählt ist aus der Gruppe bestehend aus Mineralöl, Schmiermitteln aus langen und geradketteigen Kohlenwasserstoffen Pflanzenölen und flüssigem Polymer mit einem mittleren Molekulargewicht M_{w} von 150 bis 5000 g/mol. Es ist aber auch möglich, dass das Vorlageöl ein BTL-Öl oder ein Öl ist, welches nach dem erfindungsgemäßen Verfahren hergestellt wurde, oder ein Gemisch diesen Ölen und / oder den oben genannten Ölen.

Pflanzenöle sind Gemische unterschiedlicher Acylglycerolen und enthalten weiterhin noch weitere Begleitstoffe, wie freie Fettsäuren, Phospholipide, Farbstoffe, Sterole, ätherische Öle, Vitamine, etc. In einer bevorzugten Ausführungsform handelt es sich bei den Pflanzenölen um Rapsöl und / oder um Sonnenblumenöl.

Bei der Verwendung von Mineralöl ist dieses bevorzugt ausgewählt aus der Gruppe, bestehend aus DAE (Destillated Aromatic Extracts) und / oder RAE (Residual Aromatic Extract) und / oder TDAE (Treated Destillated Aromatic Extracts) und / oder MES (Mild Extracted Solvents) und / oder naphtenische Öle, wobei TDAE besonders bevorzugt ist.

Die Erfindung soll nun anhand eines Ausführungsbeispiels näher erläutert werden. Hierzu sind in Tabelle 1 die Charakterisierungen der verwendeten Polymermaterialien und des anhand des erfindungsgemäßen Verfahrens hergestellten Weichmachers aufgeführt.

### Ausführungsbeispiel

Als Polymermaterial wird ein Gummipulver verwendet, welches aus einer vulkanisierten LKW-Laufstreifenmischung gewonnen wurde. Das Gummipulver enthält als Kautschukkomponenten hohe Anteile eines natürlichen Polyisoprens (NR), geringe Anteile von Polybutadienkautschuk (BR) und z.T. nur Spuren von Styrolbutadienkautschuk (SBR). Die in der Tabelle 1 verwendete Angabe phr (parts per hundred parts of rubber by weight) ist dabei die in der Kautschukindustrie übliche Mengenangabe für Mischungsrezepturen. Die Dosierung der Gewichtsteile der einzelnen Substanzen wird dabei stets auf 100 Gewichtsteile der gesamten Masse aller in der Mischung vorhandenen Kautschuke bezogen. Das Ausführungsbeispiel bezieht sich auf Versuche im Labormaßstab, für industrielle Anwendungen sind die entsprechenden Parameter anzupassen, insbesondere in Abhängigkeit von der entsprechenden Reaktorgröße.
Das Weichmacheröl wurde nach dem erfindungsgemäßen Verfahren hergestellt. Als Vorlageöl wurde TDAE-Öl verwendet. In einer Reaktionsapparatur von 11 Volumen wurden 302,42g TDAE-Öl (VIVATEC 500, Hansen-Rosenthal KG, Hamburg) auf 300°C erwärmt. Die Aufwärmphase beträgt 80 Minuten, um die gewünschte Sumpftemperatur von 300°C zu erreichen. Anschließend wird diese 22 Minuten gehalten, um den sogenannten Blindwert zu erfassen. Der Blindwert ist ein Produktmassenstrom, der durch das Cracken des Vorlageöles verursacht wird. Dieser Strom ist auch später bei der Zugabe des Polymermaterials vorhanden, so dass sich der gesamte Kondensatmassenstrom während des Versuches aus dem Blindwert und dem Massenstrom, der durch die Zugabe des Polymermaterials verursacht wird, zusammensetzt. Nach Ablauf der Vorwärmzeit wird die Zufuhr des Polymermaterials gestartet. Dabei ist noch zu beachten, einen Fraktionswechsel vorzunehmen, um den Einfluss des Polymermaterials auf das entstandene Kondensat besser auswerten zu können. Während der kontrollierten und kontinuierlichen Zufuhr des Polymermaterials wird das Drehmoment des Rührers in der Reaktionsapparatur beobachtet werden, um sicherzustellen, dass die Viskosität des Sumpfes nicht zu stark ansteigt. Bei temporären Viskositätsanstiegen kann kontrolliert eine kurze Pause bei der Zufuhr des Polymermaterials vorgenommen werden. Es werden im vorliegenden Ausführungsbeispiel 604,31g Polymermaterial mit einer Geschwindigkeit von 111,57 g/h in einem Zeitraum von 325 Minuten hinzugefügt. Die Temperatur während der gesamten thermischen Direktverflüssigung wird bei 300°C gehalten.
Tabelle 1 zeigt, dass keine Anteile an hochmolekularem Polymermaterial mehr vorhanden sind.

Folgende Prüfmethoden wurden verwendet, sofern nicht direkt in der Tabelle angegeben:
- Aceton-Extrakt (= Summe aller aus der vulkanisierten Gummimischung extrahierbaren Substanzen, wie bspw. Alterungsschutzmittel, Weichmacher, etc) gemäß DIN DIN ISO 308
- Rußgehalt mit TGA gemäß DIN 51006
- Glührückstand bei 550°C gemäß DIN 53568, DIN 12904, DIN 12491
- Schwefelgehalt gemäß ASTM D2622
- Glasübergangstemperatur Tg gemäß ISO DIS 28343
- Gehalt an polycyclischen Aromaten (PAK-Wert) gemäß IP 346
- Stickstoffgehalt in Anlehnung an ASTM 147
- Zn-, Cu-, Fe-Gehalt in Anlehnung an ASTM D7260

**Tabelle 1**

| **Prüfmethode** | **Einheit** | **Pulver 1** | **Weichmacheröl** |
|---|---|---|---|
| Aceton Extrakt | % | 6,4 | -- |
| Rußgehalt | Gew.-% | 28,7 | 15,2 |
| Glührückstand | % | 6 | 3,2 |
| Schwefelgehalt | % | 1,22 | 1,11 |
| Tg | °C | -62 | -17 |
| NR | phr | 83 | -- |
| BR | phr | 17 | -- |
| SBR | phr | Spuren | -- |
| PAK | % | -- | 0,12 |
| Stickstoffgehalt | % | -- | 0,23 |
| Zn | % | 1,06 | 0,58 |
| Cu | mg/kg | 1,4 | 3,5 |
| Fe | mg/kg | 24,9 | 7,6 |
| Viskosität @ RT | | n. a. | pastös |

## Patentansprüche

1. Verfahren zur Herstellung von Weichmacher, **dadurch gekennzeichnet, dass** hochmolekulares vulkanisiertes Polymermaterial durch thermische Direktverflüssigung in eine niedrigmolekulare Phase umgewandelt wird.

2. Verfahren nach Anspruch 1, **dadurch gekennzeichnet, dass** das hochmolekulare vulkanisierte Polymermaterial in Form von Polymerpulver und / oder Polymergranulat vorliegt.

3. Kautschukmischung nach Anspruch 2, **dadurch gekennzeichnet, dass** es das Polymerpulver und / oder das Polymergranulat auf der Basis von Altreifen ist.

4. Verfahren nach Anspruch 2 oder 3, **dadurch gekennzeichnet, dass** das Polymerpulver und / oder das Polymergranulat natürlichen oder synthetischen Kautschuk und / oder Butadienkautschuk und / oder Styrolbutadienkautschuk enthält.

5. Verfahren nach einem der Ansprüche 1 bis 4, **dadurch gekennzeichnet, dass** die thermische Direktverflüssigung bei Temperaturen zwischen 100 und 500°C stattfindet.

6. Verfahren nach Anspruch 5, **dadurch gekennzeichnet, dass** die thermische Direktverflüssigung bei Temperaturen zwischen 150 und 420°C stattfindet.

7. Verfahren nach einem der Ansprüche 1 bis 6, **dadurch gekennzeichnet, dass** das hochmolekulare vulkanisierte Polymermaterial in einer Sumpfphase gekrackt wird.

8. Verfahren nach einem der Ansprüche 1 bis 7, **dadurch gekennzeichnet, dass** das hochmolekulare vulkanisierte Polymermaterial einstufig gekrackt wird.

9. Verfahren nach einem der Ansprüche 1 bis 8, **dadurch gekennzeichnet, dass** für die Umwandlung des hochmolekularen vulkanisierten Polymermaterials in eine niedrigmolekulare Phase wenigstens ein Vorlageöl verwendet wird.

10. Verfahren nach Anspruch 9, **dadurch gekennzeichnet, dass** das Vorlageöl ausgewählt ist aus der Gruppe bestehend aus Mineralöl, Schmiermitteln aus langen und geradketteigen Kohlenwasserstoffen Pflanzenölen und flüssigem Polymer mit einem mittleren Molekulargewicht M_{w} von 150 bis 5000 g/mol.

## Claims

1. Process for producing plasticizer, **characterized in that** high-molecular-weight vulcanized polymer material is converted to a low-molecular-weight phase via direct thermal liquefaction.

2. Process according to Claim 1, **characterized in that** the high-molecular-weight vulcanized polymer material takes the form of polymer powder and/or granulated polymer.

3. Rubber mixture according to Claim 2, **characterized in that** it is the polymer powder and/or the granulated polymer based on used tires.

4. Process according to Claim 2 or 3, **characterized in that** the polymer powder and/or the granulated polymer comprises natural or synthetic rubber and/or butadiene rubber and/or styrene-butadiene rubber.

5. Process according to any of Claims 1 to 4, **characterized in that** the direct thermal liquefaction takes place at temperatures of from 100 to 500°C.

6. Process according to Claim 5, **characterized in that** the direct thermal liquefaction takes place at temperatures of from 150 to 420°C.

7. Process according to any of Claims 1 to 6, **characterized in that** the high-molecular-weight vulcanized polymer material in a bottom phase is cracked.

8. Process according to any of Claims 1 to 7, **characterized in that** the high-molecular-weight vulcanized polymer material is cracked in a single stage.

9. Process according to any of Claims 1 to 8, **characterized in that** for the conversion of the high-molecular-weight vulcanized polymer material to a low-molecular-weight phase at least one feed oil is used.

10. Process according to Claim 9, **characterized in that** the feed oil is one selected from the group consisting of mineral oil, lubricants made of long and short-chain hydrocarbons, vegetable oils and liquid polymer with an average molar mass M_{w} of from 150 to 5000 g/mol.

## Revendications

1. Procédé de fabrication de plastifiants, **caractérisé en ce qu'**un matériau polymère vulcanisé de poids moléculaire élevé est transformé en une phase de poids moléculaire faible par liquéfaction thermique directe.

2. Procédé selon la revendication 1, **caractérisé en ce que** le matériau polymère vulcanisé de poids moléculaire élevé se présente sous la forme d'une poudre de polymère et/ou d'un granulat de polymère.

3. Mélange de caoutchouc selon la revendication 2, **caractérisé en ce qu'**il est la poudre de polymère et/ou le granulat de polymère à base de pneus usagés.

4. Procédé selon la revendication 2 ou 3, **caractérisé en ce que** la poudre de polymère et/ou le granulat de polymère contiennent un caoutchouc naturel ou synthétique et/ou un caoutchouc de butadiène et/ou un caoutchouc de styrène-butadiène.

5. Procédé selon l'une quelconque des revendications 1 à 4, **caractérisé en ce que** la liquéfaction thermique directe a lieu à des températures comprises entre 100 et 500 °C.

6. Procédé selon la revendication 5, **caractérisé en ce que** la liquéfaction thermique directe a lieu à des températures comprises entre 150 et 420 °C.

7. Procédé selon l'une quelconque des revendications 1 à 6, **caractérisé en ce que** le matériau polymère vulcanisé de poids moléculaire élevé est craqué dans une phase de fond.

8. Procédé selon l'une quelconque des revendications 1 à 7, **caractérisé en ce que** le matériau polymère vulcanisé de poids moléculaire élevé est craqué en une étape.

9. Procédé selon l'une quelconque des revendications 1 à 8, **caractérisé en ce qu'**au moins une huile support est utilisée pour la transformation du matériau polymère vulcanisé de poids moléculaire élevé en une phase de poids moléculaire faible.

10. Procédé selon la revendication 9, **caractérisé en ce que** l'huile support est choisie dans le groupe constitué par une huile minérale, les lubrifiants à base d'hydrocarbures à chaînes longues et linéaires, les huiles végétales et un polymère liquide ayant un poids moléculaire moyen M_{w} de 150 à 5 000 g/mol.
